(19) Europäisches Patentamt
European Patent Office
Office européen des brevets

(11) Veröffentlichungsnummer: **0 260 644 B1**

(12) **EUROPÄISCHE PATENTSCHRIFT**

(45) Veröffentlichungstag der Patentschrift: **06.11.91**

(51) Int. Cl.[5]: **C07D 207/22**, B01D 53/14

(21) Anmeldenummer: **87113435.9**

(22) Anmeldetag: **15.09.87**

# (54) Verfahren zur Herstellung von alpha-Pyrrolidonen und deren Iminen.

(30) Priorität: **16.09.86 DE 3631414**

(43) Veröffentlichungstag der Anmeldung:
**23.03.88 Patentblatt 88/12**

(45) Bekanntmachung des Hinweises auf die Patenterteilung:
**06.11.91 Patentblatt 91/45**

(84) Benannte Vertragsstaaten:
**BE CH DE ES FR GB IT LI NL**

(56) Entgegenhaltungen:
**AU-B- 67 247**
**DE-C- 694 043**
**US-A- 4 208 382**
**DE-B- 1 795 007**
**DE-C- 861 845**
**US-A- 4 460 385**

(73) Patentinhaber: **BASF Aktiengesellschaft**
**Carl-Bosch-Strasse 38**
**W-6700 Ludwigshafen(DE)**

(72) Erfinder: **Wambach, Ludwig, Dr.**
**Humboldtstrasse 28**
**W-6900 Heidelberg(DE)**

**EP 0 260 644 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von α-Pyrrolidonenund deren Iminen.

Es ist bekannt, daß Amine mit α,β-ungesättigten Carbonsäurederivaten oder α,β-ungesättigten Nitrilen sehr leicht eine Reaktion vom Typ der Michael-Addition eingehen, wobei offenkettige, gesättigte Verbindungen entstehen.

α-Pyrrolidone wurden bisher in der Weise hergestellt, daß man Lactone mit Ammoniak oder Aminen umsetzt, vgl. DE-A-1 795 007. Dieses Verfahren ist verhältnismäßig kostspielig, weil die Lactone im allgemeinen erst hergestellt werden müssen. Auch andere Wege, wie beispielsweise über die entsprechenden Nitrocarbonsäuren, wie in Org. Synth. 32, 59 (1952) beschrieben ist, sind wesentlich aufwendiger, vgl. DE-A-861 845.

Der Erfindung lag die Aufgabe zugrunde, ein neues Verfahren zur Herstellung von α-Pyrrolidonen und deren Iminen der allgemeinen Formeln Ia und Ib

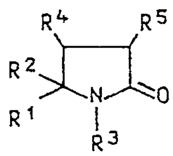
(Ia)

.

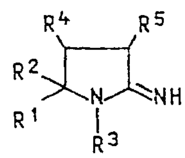
(Ib)

in denen $R^1$ und $R^2$ für $C_1$-$6_8$-Alkyl, $C_1$-$C_8$-Alkoxy, $C_3$-$C_{10}$-Cycloalkyl oder $C_3$-$C_{10}$-Cycloalkyloxy stehen, wobei $R^1$ und $R^2$ gemeinsam einen Ring mit insbesondere 4 bis 7 Ringkohlenstoffatomen bilden können, $R^3$ Wasserstoff, $C_1$-$C_6$-Alkyl, Phenyl oder Naphthyl bedeutet und $R^4$ und $R^5$ für Wasserstoff oder $C_1$-$C_4$-Alkyl stehen,

bereitzustellen, das die Herstellung der gewünschten Verbindungen in einfacher und kostengünstiger Weise erlaubt.

Diese Aufgabe wird dadurch gelöst, daß man ein Amin der allgemeinen Formel II

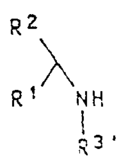
(II)

in der $R^{3'}$ Wasserstoff, tert. -Butyl, Phenyl oder Naphthyl bedeutet, mit einem α,β-ungesättigten Carbonsäurederivat der allgemeinen Formel III

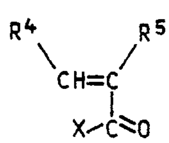
(III)

in der X für -OR′, -NR′R″, -NHR′ oder $-NH_2$ steht, wobei die Reste R′ und R″ $C_1$-$C_8$-Alkyl, $C_3$-$C_8$-Cycloalkyl oder Phenyl bedeuten, bzw.

mit einem α,β-ungesättigten Nitril der allgemeinen Formel IV

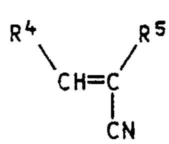
(IV)

in Gegenwart eines Radikalstarters umsetzt und die hierbei erhältlichen Verfahrensprodukte für den Fall, daß $R^{3'}$ Wasserstoff bedeutet und Verbindungen mit $R^3$ = $C_1$-$C_6$-Alkyl hergestellt werden sollen, mit einem Alkylierungsmittel zur Einführung einer $C_1$-$C_6$-Alkylgruppe umsetzt.

Nach einer bevorzugten Ausführungsform des erfindungsgemäßen Verfahrens verwendet man als Radikalstarter Alkylperester, Dialkylperoxide, Diarylperoxide oder Azoverbindungen. Allgemein können als Radikalstarter alle Peroxide und Azoverbindungen verwendet werden, welche zur Radikalbildung geeignet sind und mit Aminen keine spontane Reaktion eingehen. Besonders geeignet sind Azo-bis-isobutyronitril und 2,2'-Azo-bis-(2,4-dimethylvaleronitril). Weiterhin ist besonders bevorzugt die Verwendung von Di-tert.-butylperoxid, wobei man zweckmäßig bei erhöhten Temperaturen, beispielsweise im Bereich von etwa 100 bis 200 °C, insbesondere 150 bis 160 °C, arbeitet. Die Wahl des Radikalstarters richtet sich allgemein nach der Reaktionstemperatur, die vorzugsweise im Bereich von 50 bis 200 °C liegt. Besonders bevorzugt wählt man als Reaktionstemperatur die Siedetemperatur des Reaktionsgemischs.

Nach weiteren bevorzugten Ausführungsformen führt man die Reaktion bei Normaldruck oder erhöhtem Druck durch. Drücke von 1 bis 200 bar sind besonders bevorzugt. Zweckmäßig arbeitet man im Autoklaven bei erhöhter Temperatur unter einem Druck von insbesondere 1 bis 50 bar.

Es ist häufig zweckmäßig, einen Überschuß aus Amin (II) einzusetzen, da dieses zugleich als Lösungsmittel dient. Das Verhältnis von Amin (II) zum $\alpha,\beta$-ungesättigten Carbonsäurederivat (III) bzw. dem entsprechenden Nitril (IV) beträgt zweckmäßigerweise 1 : 1 bis 10 : 1.

Die Menge des Radikalstarters kann in weiten Grenzen variiert werden. Aus wirtschaftlichen Gründen vorteilhaft sind 5 bis 20 mol% Radikalstarter, bezogen auf die $\alpha,\beta$-ungesättigte Komponente (III) bzw. (IV).

Gegenstand der Erfindung sind auch neue $\alpha$-Pyrrolidone der allgemeinen Formel Ia'

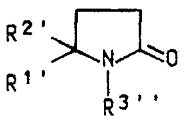

(Ia')

worin $R^{1'}$ und $R^{2'}$ für $C_1$-$C_4$-Alkyl, insbesondere Methyl, stehen oder gemeinsam mit dem C-Atom, an das sie gebunden sind, einen Ring, insbesondere einen carbocyclischen Ring mit insbesondere 4 bis 7 Ringkohlenstoffatomen bilden können und worin $R^{3''}$ Methyl oder tert.-Butyl bedeutet.

Die Reste $R^1$ und $R^2$ stehen für $C_1$-$C_8$-Alkyl, insbesondere für $C_1$-$C_4$-Alkyl, insbesondere für Methyl, Ethyl, n-Propyl, iso-Propyl und n-Butyl. $R^1$ und $R^2$ können auch für $C_1$-$C_8$-Alkoxy stehen, insbesondere für $C_1$-$C_4$-Alkoxy, beispielsweise Methoxy, Ethoxy, Propoxy. $R^1$ und $R^2$ können auch $C_3$-$C_{10}$-Cycloalkyl, insbesondere $C_3$-$C_6$-Cycloalkyl, beispielsweise Cyclopropyl, Cyclobutyl, Cyclopentyl, Cyclohexyl, bedeuten. $R^1$ und $R^2$ können auch für einen $C_3$-$C_{10}$-Cycloalkyloxyrest, insbesondere einen $C_3$-$C_6$-Cycloalkyloxyrest, stehen. Die genannten Gruppen können gewünschtenfalls weiter substituiert sein, so beispielsweise durch $C_1$-$C_4$-Alkyl, Halogen und $C_1$-$C_4$-Alkoxy.

Der Rest $R^3$ bedeutet Wasserstoff oder eine $C_1$-$C_6$-Alkyl-, Phenyl-oder Naphthylgruppe.

Die Reste $R^4$ und $R^5$ können für Wasserstoff oder $C_1$-$C_4$-Alkyl, beispielsweise Methyl, Ethyl, n-Propyl, Isopropyl, Butyl stehen.

In den Verbindungen (III) kann der Rest X für -OR', -NR'R", -NHR' oder -$NH_2$ stehen, wobei die Reste R' und R" für $C_1$-$C_8$-Alkyl, insbesondere $C_1$-$C_4$-Alkyl, $C_3$-$C_8$-Cycloalkyl, insbesondere $C_3$-$C_6$-Cycloalkyl oder Phenyl, stehen. Falls X für -NR'R", NHR oder $NH_2$ steht, ist es gegebenenfalls erforderlich, stärker zu erhitzen, um sofort das entsprechende $\alpha$-Pyrrolidon zu erhalten.

Als Amine (II) sind Isopropylamin, Cyclohexylamin, Cyclopentylamin, sec.-Butylamin, N-Isopropyl-t-butylamin und N-Isopropylanilin besonders geeignet. Sollen Verbindungen I hergestellt werden, in denen $R^3 \neq R^{3'}$ ist, ist ein derartiger Rest durch nachträgliche Alkylierung einer Verbindung I mit $R^3$ = H herzustellen, beispielsweise durch Umsetzung mit einem entsprechenden Alkanol oder Alkylbromid in an sich bekannter Weise.

Als $\alpha,\beta$-ungesättigte Carbonsäurederivate (III) bzw. (IV) sind Acrylsäureester, Crotonsäureester, Acrylsäureamide und Acrylsäurenitrile besonders bevorzugt.

Nach einer besonders bevorzugten Ausführungsform setzt man Isopropylamin mit Acrylsäuremethylester in Gegenwart von Di-tert.-butylperoxid zum 5,5-Dimethylpyrrolidon-2 um.

Zweckmäßigerweise wird das erfindungsgemäße Verfahren so durchgeführt, daß die Hauptmenge Amin vorgelegt und auf die gewünschte Temperatur erhitzt wird. Den Radikalstarter verdünnt man mit der Restmenge Amin und dosiert diese Lösung zeitgleich mit der $\alpha,\beta$-ungesättigten Komponente zu. Radikalstarter, welche mit der $\alpha,\beta$-ungesättigten Komponente bei Raumtemperatur keine Reaktion eingehen,

können auch mit dieser vermischt und zudosiert werden.

Aus dem Reaktionsgemisch lassen sich die Verbindungen Ia bzw. Ib mit Hilfe üblicher Trennmethoden, vorzugsweise durch Destillation, leicht isolieren. Nimmt man die Aufarbeitung im Falle der Verbindungen Ib in Gegenwart von Wasser vor, erhält man die entsprechenden Pyrrolidone Ia.

Die α-Pyrrolidone und deren Imine Ia und Ib sind wertvolle Zwischenprodukte, insbesondere für pharmazeutische Zwecke. Durch N-Alkylierung der N-unsubstituierten Pyrrolidone Ia mit $C_1$-$C_6$-Alkanolen, beispielsweise unter Anwendung der Arbeitsweise gemäß DE-A-830 194, erhält man aus di e N-Alkylpyrrolidone Ia, welche als Lösungsmittel, z.B. zur Gaswäsche, eingesetzt werden können. Unter derartigen N-Alkylpyrrolidonen ist das N-Methyl-5,5-dimethylpyrrolidon-2 ein besonders geeignetes Lösungsmittel für die Gaswäsche zur Entfernung saurer Gase wie Kohlendioxid oder Schwefelwasserstoff aus Gasgemischen. Hierbei bieten die erfindungsgemäßen Verbindungen den Vorteil der Oxidationsunempfindlichkeit, d.h. man kann sie besonders erfolgreich zur Wäsche sauerstoffhaltiger Gase verwenden.

Beispiel 1

In einem 2,5 l-Autoklaven wurden 575 g (9,7 mol) Isopropylamin vorgelegt. Dann wurden bei 150 °C innerhalb von zwei Stunden 300 g (3,5 mol) Acrylsäuremethylester und 15 g (0,1 mol) Di-tert.-Butylperoxid, gelöst in 215 g (3,65 mol) Isopropylamin, zudosiert. Die Nachrührzeit betrug zwei Stunden. Die destillative Aufarbeitung lieferte das 5,5-Dimethylpyrrolidon-2 (Siedepunkt: 85 - 87 °C bei 0,5 mbar) in 80 %iger Ausbeute.

Beispiel 2

Analog Beispiel 1 wurden 7,8 mol Cyclohexylamin und 30 g (0,2 mol) Di-tert.-Butylperoxid in 260 g (2,6 mol) Cyclohexylamin zum 5-Spiro-cyclohexyl-pyrrolidon-2 umgesetzt; Ausbeute 70 %; F: 132 - 133 °C; Siedepunkt: 155 - 158 °C bei 0,5 mbar.

Beispiel 3

8 mol Cyclohexylamin wurden bei 80 °C allmählich mit 3 mol Acrylsäuremethylester und 37 g (0,1 mol) trockenem Dilauroylperoxid versetzt. Anschließend wurde noch drei Stunden bei 80 °C nachgerührt. Die destillative Aufarbeitung lieferte das 5-Spiro-cyclohexylpyrrolidon-2 in 70 %iger Ausbeute.

Beispiel 4

Analog Beispiel 1, jedoch mit 300 g (3 mol) Crotonsäuremethylester anstelle des Acrylsäuremethylesters wurde das 4,5,5-Trimethylpyrrolidon-2 in 30%iger Ausbeute (Siedepunkt: 98 - 100 °C bei 0,3 mbar) hergestellt.

Beispiel 5

Analog Beispiel 4, jedoch mit 57,3 g (0,2 mol) 2,5-Dimethyl-2,5-di-tert.-butylperoxy-3-hexin als Radikalstarter sowie einer Reaktionstemperatur von 160 °C fiel das 4,5,5-Trimethylpyrrolidon-2 in 45 %iger Ausbeute an.

Beispiel 6

Analog Beispiel 1, jedoch mit 342,5 g (3,0 mol) Methacrylsäureethylester anstelle des Acrylsäuremethylesters wurde das 3,5,5-Trimethylpyrrolidon-2 in 15 % Ausbeute (Siedepunkt: 103 - 107 °C bei 0,3 mbar) hergestellt.

Beispiel 7

354,7 g (6 mol) Isopropylamin wurden vorgelegt und bei 150 °C innerhalb von zwei Stunden mit 213,24 g (3 mol) Acrylsäureamid, gelöst in 213 g Methanol, und 43,87 g (0,3 mol) Di-tert.-butylperoxid, gelöst in 177,35 g (3 mol) Isopropylamin, versetzt. Die Nachrührzeit betrug zwei Stunden. Die anschließende destillative Aufarbeitung lieferte das 5,5-Dimethylpyrrolidon-2 in 50 %iger Ausbeute.

Beispiel 8

575 g (9,7 mol) Isopropylamin wurden analog Beispiel 1 mit 189 g (3,5 mol) Acrylnitril umgesetzt, wobei sich das 5,5-Dimethylpyrrolidon-2-imin gemäß GC-Analyse in rd. 70 %iger Ausbeute bildete.

Beispiel 9

Eine Lösung aus 113 g (1 mol) 5,5-Dimethyl-pyrrolidon-2 und 96 g (3 mol) Methanol wurde verdampft und bei 400 °C über einen Aluminiumoxid-Kontakt geleitet. Die übliche Aufarbeitung des Gasgemisches auf das 1,5,5-Trimethylpyrrolidon-2 lieferte diese Verbindung in rd. 86 %iger Ausbeute; Sdp. 236-238 °C.

**Patentansprüche**

**1.** Verfahren zur Herstellung von α-Pyrrolidonen und deren Iminen der allgemeinen Formeln Ia und Ib

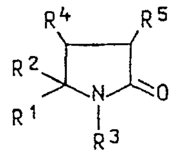

(Ia)

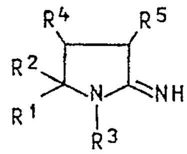

(Ib)

in denen $R^1$ und $R^2$ für $C_1$-$C_8$-Alkyl, $C_1$-$C_8$-Alkoxy, $C_3$-$C_{10}$-Cycloalkyl oder $C_3$-$C_{10}$-Cycloalkyloxy stehen, wobei $R^1$ und $R^2$ gemeinsam einen Ring mit insbesondere 4 bis 7 Ringkohlenstoffatomen bilden können, $R^3$ Wasserstoff, $C_1$-$C_6$-Alkyl, Phenyl oder Naphthyl bedeutet, und $R^4$ und $R^5$ für Wasserstoff oder $C_1$-$C_4$-Alkyl stehen,

dadurch gekennzeichnet, daß man ein Amin der allgemeinen Formel II

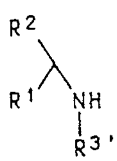

(II)

in der $R^{3'}$ Wasserstoff, tert.-Butyl, Phenyl oder Naphthyl bedeutet,

mit einem α,β-ungesättigten Carbonsäurederivat der allgemeinen Formel III

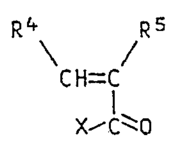

(III)

in der X für -OR' , -NR'R" , -NHR' oder $-NH_2$ steht, wobei die Reste R' und R" $C_1$-$C_8$-Alkyl, $C_3$-$C_8$-Cycloalkyl oder Phenyl bedeuten, bzw.

mit einem α,β-ungesättigten Nitril der allgemeinen Formel IV

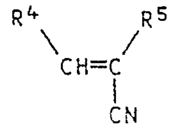

(IV)

in Gegenwart eines Radikalstarters umsetzt und die hierbei erhältlichen Verfahresprodukte für den Fall, daß $R^{3'}$ Wasserstoff bedeutet und Verbindungen mit $R^3$ = $C_1$-$C_6$-Alkyl hergestellt werden sollen, mit einem Alkylierungsmittel zur Einführung einer $C_1$-$C_6$-Alkyl-gruppe umsetzt.

**2.** Verfahren nach Anspruch 1 , dadurch gekennzeichnet, daß man als Radikalstarter Alkylperester, Dialkylperoxide, Diarylperoxide oder Azoverbindungen einsetzt.

**3.** Verfahren nach den Ansprüchen 1 und 2, dadurch gekennzeichnet, daß man bei der Umsetzung von (III) mit (III) bzw. (IV) bei Temperaturen von 50 bis 200° C arbeitet.

**4.** Verfahren nach den Ansprüchen 1 bis 3, dadurch gekennzeichnet, daß man bei der Umsetzung von (II) mit (III) bzw. (IV) bei Drücken von 1 bis 200 bar arbeitet.

**5.** Verfahren nach den Ansprüchen 1 bis 4, dadurch gekennzeichnet, daß man bei der Umsetzung von (II) mit (III) bzw. (IV) in Gegenwart eines Überschusses an dem Amin II als Lösungsmittel arbeitet.

**6.** α-Pyrrolidone der allgemeinen Formel Ia'

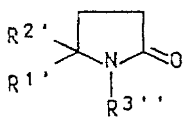

(Ia')

worin $R^{1'}$ und $R^{2'}$ für $C_1$-$C_4$-Alkyl stehen oder gemeinsam mit dem C-Atom, an das sie gebunden sind, einen Ring mit insbesondere 4 bis 7 Ringkohlenstoffatomen bilden können und worin $R^{3''}$ Methyl oder tert. -Butyl bedeutet.

**7.** α- Pyrrolidone der allgemeinen Formel Ia' gemäß Anspruch 6, worin die Reste $R^{1'}$ , $R^{2'}$ und $R^{3'}$ jeweils für Methyl stehen.

## Claims

**1.** A process for the preparation of an α-pyrrolidone or its imine of the formula Ia or Ib

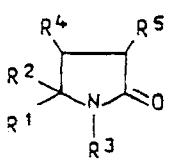

(Ia)

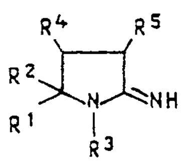

(Ib)

where $R^1$ and $R^2$ are each $C_1$-$C_8$-alkyl, $C_1$-$C_8$-alkoxy, $c_3$-$c_{10}$-cycloalkyl or $C_3$-$C_{10}$-cycloalkoxy and $R^1$ and $R^2$ together may form a ring having, in particular, from 4 to 7 ring carbon atoms, $R^3$ is hydrogen, $C_1$-$C_6$-alkyl, phenyl or naphthyl and $R^4$ and $R^5$ are each hydrogen or $C_1$-$C_4$-alkyl, wherein an amine of the formula II

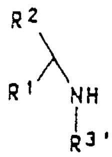

(II)

where $R^{3'}$ is hydrogen, tert-butyl, phenyl or naphthyl, is reacted with an $\alpha,\beta$-unsaturated carboxylic acid derivative of the formula III

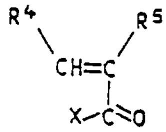

(III)

where X is -OR', -NR'R'', -NHR' or $-NH_2$, in which R' and R'' are each $C_1$-$C_8$-alkyl, $C_3$-$C_8$-cycloalkyl or phenyl, or with an $\alpha,\beta$-unsaturated nitrile of the formula IV

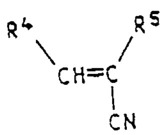

(IV)

in the presence of a free radical initiator, and, where $R^{3'}$ is hydrogen and it is intended to prepare a compound in which $R^3$ is $C_1$-$C_4$-alkyl, the product obtainable in this procedure is reacted with an alkylating agent to introduce a $C_1$-$C_6$-alkyl group.

2. A process as claimed in claim 1, wherein the free radical initiator used is an alkyl perester, a dialkyl peroxide, a diaryl peroxide or an azo compound.

3. A process as claimed in claim 1 or 2, wherein the reaction of (II) with (III) or (IV) is carried out at from 50 to 200° C.

4. A process as claimed in any of claims 1 to 3, wherein the reaction of (II) with (III) or (IV) is carried out under from 1 to 200 bar.

5. A process as claimed in any of claims 1 to 4, wherein the reaction of (II) with (III) or (IV) is carried out in the presence of an excess of the amine II as a solvent.

6. An $\alpha$-pyrrolidone of the formula Ia'

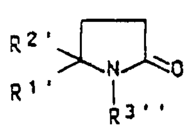

(Ia')

where $R^{1'}$ and $R^{2'}$ are each $C_1$-$C_4$-alkyl or, together with the carbon atom to which they are bonded, may form a ring having, in particular, from 4 to 7 ring carbon atoms, and $R^{3''}$ is methyl or tert-butyl.

7. An $\alpha$-pyrrolidone of the formula Ia' as claimed in claim 6, wherein $R^{1'}$, $R^{2'}$, $R^{3'}$ are each methyl.

**Revendications**

1. Procédé de préparation d'α-pyrrolidones et de leurs imines de formules générales Ia et Ib

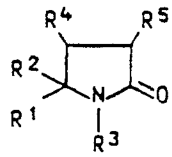 (Ia) 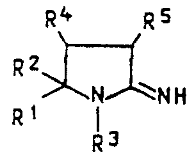 (Ib)

dans lesquelles $R^1$ et $R^2$ sont mis pour des radicaux alkyle en $C_1$-$C_8$, alcoxy en $C_1$-$C_8$, cycloalkyle en $C_3$-$C_{10}$ ou cycloalcoxy en $C_3$-$C_{10}$, $R^1$ et $R^2$ pouvant former ensemble un noyau comportant en particulier de 4 à 7 atomes de carbone nucléaires,
$R^3$ est un atome d'hydrogène ou un radical alkyle en $C_1$-$C_6$,phényle ou naphtyle, et
$R^4$ et $R^5$ sont mis chacun pour un atome d'hydrogène ou un radical alkyle en $C_1$-$C_4$,
caractérisé en ce qu'on fait réagir, en présence d'un initiateur radicalaire, une amine de formule générale II

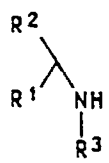 (II)

dans laquelle $R^{3'}$ représente un atome d'hydrogène ou un radical butyle tertiaire, phényle ou naphtyle, avec un dérive d'acide carboxylique α,β-insaturé de formule générale III

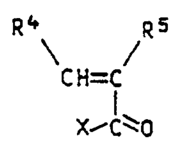 (III)

dans laquelle X est mis pour -OR', -NR'R" -NHR' ou $-NH_2$, les restes R' et R" etant des radicaux alkyle en $C_1$-$C_8$, cycloalkyle en $C_3$-$C_8$ ou phenyle, ou respectivement
avec un nitrile α,β-insaturé de formule générale IV

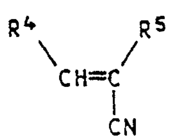 (IV)

et, dans le cas ou $R^{3'}$ represente un atome d'hydrogène et ou l'on veut obtenir des composes dans lesquels $R^3$ = alkyle en $C_1$-$C_6$, on fait réagir les produits réactionnels ainsi obtenus avec un agent d'alkylation pour l'introduction d'un groupement alkyle en $C_1$-$C_6$.

2. Procédé selon la revendication 1, caractérisé en ce qu'on utilise, comme initiateurs radicalaires, des peresters d'alkyle, des peroxydes de dialkyle, des peroxydes de diaryle ou des composés azoïques.

3. Procédé selon la revendication 1 ou 2, caractérisé en ce qu'on conduit la réaction de (II) avec (III) ou avec (IV) à des températures de 50 à 200°C .

**4.** Procédé selon l'une quelconque des revendications 1 à 3, caractérisé en ce qu'on conduit la réaction de (II) avec (III) ou avec (IV) sous des pressions de 1 à 200 bar.

**5.** Procédé selon l'une quelconque des revendications 1 à 4, caractérisé en ce qu'on conduit la réaction de (II) avec (III) ou avec (IV) en présence d'un exces de l'amine il servant de solvant.

**6.** α-Pyrrolidones de formule générale Ia'

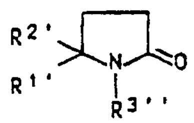

(Ia')

dans laquelle R'' et $R^{2'}$ sont mis pour des radicaux alkyle en $C_1$-$C_4$ ou peuvent former, avec l'atome de carbone auquel ils sont fixés, un noyau comportant en particulier de 4 à 7 atomes de carbone nucléaires, et dans laquelle $R^{3''}$ représente un radical methyle ou tert.-butyle.

**7.** α-Pyrrolidones de formule générale Ia' selon la revendication 6, dans laquelle les restes R'', $R^{2'}$ et $R^{3'}$ sont mis chacun pour un radical méthyle.